(19)

Europäisches
Patentamt
European
Patent Office
Office européen
des brevets

(11)  **EP 2 250 964 A1**

(12)  **EUROPEAN PATENT APPLICATION**

(43) Date of publication:
**17.11.2010 Bulletin 2010/46**

(51) Int Cl.:
**A61B 5/11** (2006.01)     **A63B 24/00** (2006.01)
**A61B 5/103** (2006.01)

(21) Application number: **10161185.3**

(22) Date of filing: **27.04.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA ME RS**

(30) Priority: **12.05.2009 JP 2009115720**

(71) Applicant: **Oki Electric Industry Co., Ltd.**
**Minato-ku**
**Tokyo**
**105-8460 (JP)**

(72) Inventors:
• **Okuya, Daisuke**
**Tokyo 105-8460 (JP)**
• **Maeno, Kurato**
**Tokyo 105-8460 (JP)**

(74) Representative: **Schöniger, Franz-Josef**
**Betten & Resch,**
**Patentanwälte,**
**Theatinerstrasse 8**
**80333 München (DE)**

(54)  **Motion analysis device, program and method thereof and motion analysis system**

(57)    The present invention relates to a motion analysis system that includes a sensor device for measuring a motion amount related to an object which repeats a certain motion and a motion analysis device for analyzing the certain motion of the object based on the measured result by the sensor device. The motion analysis device includes means for storing time series data of the motion amount measured by the sensor device; means for analyzing a spectral intensity in each frequency in time series of waveforms shown by the time series data by using a certain function and analyzing by applying different parameters for each frequency to the function; and means for comparing spectral intensity analysis result in each frequency with different frequencies and analyzing a certain physical amount related to the certain motion of the object based on the comparison result.

FIG.1

EP 2 250 964 A1

**Description**

BACKGROUND OF THE INVENTION

Field of the Invention

The present invention relates to a motion analysis device, a program and a method thereof and a motion analysis system.

Description of the Related Art

A method for detecting a pace of motion or a motion amount with a motion sensor attached to a human body has been widely proposed in purpose of recognition of a condition of a human in a health condition management, rehabilitation, or the like.

In JP-A No. 2006-118909, JP-A No. 2005-233771, JP-A No. 2005-342254, and JP-A No. 2004-121539, an acceleration sensor is attached to a user's lumbar part, leg or arm and waveforms in time series changes of the acceleration obtained during walking activity are converted into frequency components by Fourier transform, and the pace of motion or the like are calculated by assuming that the frequency in which the spectral intensity is in peak as one cycle of motion (in the case of walking, two steps is one cycle when the sensor is attached to the lumbar part and one step is one cycle when the sensor is attached to a leg) and the spectral intensity thereof as a level of motion.

Further, JP-A No. 2005-49202, JP-A No. 2008-154733, and JP-A No. 2006-101973 disclose a technology that focuses on the waveform of each motion cycle and obtains the length of motion cycle as an index indicating a pace by using a method of a peak value interval, zero-crossing or the like.

SUMMARY OF THE INVENTION

However, the all above documents focus on walking whose one motion cycle is around 1 to 3.5 Hz as the motion to be analyzed. It is difficult to use the technology for motion having motion cycles longer than walking activity or the shape of acceleration waveforms in one cycle is complex, which includes, for example, lifting dumbbells or exercising using a weight training equipment at a gym or lifting something or operating machines in daily life. To achieve an object to manage human's conditions such as a health condition management, rehabilitation or workers' operations, various motions in addition to walking activities have to be handled.

In a method based on Fourier transform, including the technologies disclosed in JP-A No. 2006-118909, JP-A No. 2005-233771, JP-A No. 2005-342254 and JP-A No. 2004-121539, a frequency region of a pace to be obtained is up to a half of sampling rate (Nyquist frequency) that a sensor obtains data and the resolution depends on the sampling rate that the sensor obtains data and the time window used in Fourier transform. There is a need to lower the sampling rate of the sensor or extend the time window of Fourier transform to obtain a higher pace resolution.

In general, the pace of walking is not relatively too low compared to the sampling rate of the sensor and the resolution using Fourier transform can sufficiently distinguish the difference in paces of walking. However, to distinguish paces of motion Slower than walking with a high resolution, there is a need to lower the sampling rate of the sensor or repeat a certain motion for a long period of time. To lower the sampling rate, information of motion is truncated and repeating the same motion for a long period of time cannot be a practical solution.

Further, motion slower than walking action may include various types of actions and the amplitude, cycle or shapes of waveforms obtained by the sensor for each motion differs.

In a method using the shapes of waveforms obtained by the sensor as disclosed in JP-A No. 2005-49202, JP-A No. 2008-154733 and JP-A No. 2006-101973, it is desirable to accurately distinguish the waveforms in a cycle of motion and preprocessing such as a reduction of noise component by using a low-pass filter is important for exaggerating the shape of waveforms indicating motion. However, it is difficult to obtain a proper peak value or zero-crossing from complex waveforms without the preprocessing. Thus, it is difficult to uniformly obtain a pace from various motions.

Thus, a motion analysis device, a program and a method thereof and a motion analysis system which are capable of analyzing at a high accuracy with a least processing amount in an analysis related to motion of a subject such as a person or an object has been desired.

According to an embodiment of the present invention, there is provided a motion analysis device constituting (1) a motion analysis system that includes a sensor device for measuring a motion amount related to motion of an object which repeats a certain motion and the motion analysis device for analyzing the certain motion of the object based on the measured result of the sensor device, the motion analysis device including: (2) time series data storing section configured to store time series data of the motion amount measured by the sensor device; (3) waveform analyzing section configured to analyze a spectral intensity in each frequency by applying a certain function to a waveform shown by the time series data and analyze by applying different parameters for each frequency to the function; and (4) motion analysis section configured to compare, for different frequencies, the spectral intensity analysis result in each frequency analyzed by the waveform analyzing section and analyze a certain physical amount of the certain motion of the object by comparing based on the comparison result.

According to another embodiment of the present invention, there is provided a motion analysis program that controls (1) a computer mounted in a motion analysis device constituting a motion analysis system that includes a sensor device for measuring a motion amount related to motion of an object which repeats a certain motion and the motion analysis device for analyzing the certain motion of the object based on the measured result by the sensor device, the motion analysis program that controls the computer to function as: (2) time series data storing section configured to store time series data of the motion amount measured by the sensor device; (3) waveform analyzing section configured to analyze the spectral intensity in each frequency by applying a certain function to a waveform shown by the time series data and analyze by applying different parameters for each frequency to the function; and (4) motion analysis section configured to compare for different frequencies, the spectral intensity analysis result in each frequency analyzed by the waveform analyzing section and analyze a certain physical amount of the certain motion of the object by comparing based on the comparison result.

According to another embodiment of the present invention, there is provided a motion analysis method (1) for analyzing a certain motion of an object based on a measured result by a sensor device for measuring a motion amount related to the motion of the object which repeats the certain motion, including (2) time series data storing section, waveform analyzing section and motion analysis section, wherein (3) the time series data storing section stores time series data of the motion amount measured by the sensor device, (4) the waveform analyzing section analyzes the spectral intensity in each frequency by applying a certain function to a waveform shown by the time series data and analyzes by applying different parameters for each frequency to the function, and (5) the motion analysis section compares for different frequencies, the spectral intensity analysis result in each frequency analyzed by the waveform analyzing section and analyzes a certain physical amount of the certain motion of the object by comparing based on the comparison result.

According to another embodiment of the present invention, there is provided a motion analysis system that includes (1) a sensor device for measuring a motion amount related to motion of an object which repeats a certain motion and the motion analysis device for analyzing the certain motion of the object based on the measured result by the sensor device, wherein (2) the motion analysis device according to the first invention is applied as the motion analysis device.

According to the present invention, an analysis at a high accuracy with a least processing amount can be achieved in an analysis related to motion of a subject such as a person or an object.

BRIEF DESCRIPTION OF THE DRAWINGS

FIG. 1 is a block diagram showing an entire configuration of a motion analysis system according to a first embodiment;
FIG. 2 is an explanatory view showing an example of information stored in a range information storage unit according to the first embodiment;
FIG. 3 is a flowchart showing an operation related to an analysis in a motion analysis device according to the first embodiment;
FIG. 4 is a graph showing an example of waveforms indicated by time series data of a motion amount measured in a sensing device according to the first embodiment;
FIG. 5 is a graph showing a result of a wavelet transform in a calculating unit according to the first embodiment;
FIG. 6 is an explanatory view showing a manner of further clipping data from the result of the wavelet transform calculated in the calculating unit according to the first embodiment;
FIG. 7 is a graph showing spectral intensity cumulative values calculated in the calculating unit according to the first embodiment;
FIG. 8 is an explanatory view showing a manner of analyzing a motion pace of a moving user in the calculating unit according to the first embodiment;
FIG. 9 is a block diagram showing an entire configuration of a motion analysis system according to a second embodiment;
FIG. 10 is an explanatory view showing an example of information stored in a motion amount correspondence storage unit according to the second embodiment;
FIG. 11 is an explanatory view showing a manner of analyzing a relationship of a motion pace spectrum and a load amount of a weight training equipment in a motion amount conversion unit according to the second embodiment;
FIG. 12 is a flowchart showing an operation related to an analysis in a motion analysis device according to the second embodiment;

FIG. 13 is a block diagram of an entire configuration of a motion analysis system according to a third embodiment;

FIG. 14 is a graph showing a result of a wavelet transform based on time series motion data in a case where an abnormal action occurs during a measurement by a sensing device in the motion analysis system according to the third embodiment;

FIG. 15 is a graph in a case where a spectral intensity cumulative value in each frequency in a time window including a zone in which an abnormal action occurs is calculated in a calculating unit according to the third embodiment;

FIG. 16 is a graph in a case where a spectral intensity cumulative value in each frequency in a time window including a zone in which an abnormal action occurs is calculated in the calculating unit according to the third embodiment;

FIG. 17 is an explanatory view showing a manner of detecting an occurrence of an abnormal action in an abnormal action detection unit according to the third embodiment; and

FIG. 18 is a flowchart showing an operation related to an analysis in a motion analysis device according to the third embodiment.

DETAILED DESCRIPTION OF THE EMBODIMENTS

(A) First embodiment

Hereinafter, a first embodiment of a motion analysis device, a program and a method thereof and a motion analysis system according to the present invention will be described in detail with reference to the drawings.

(A-1) Configuration of first embodiment

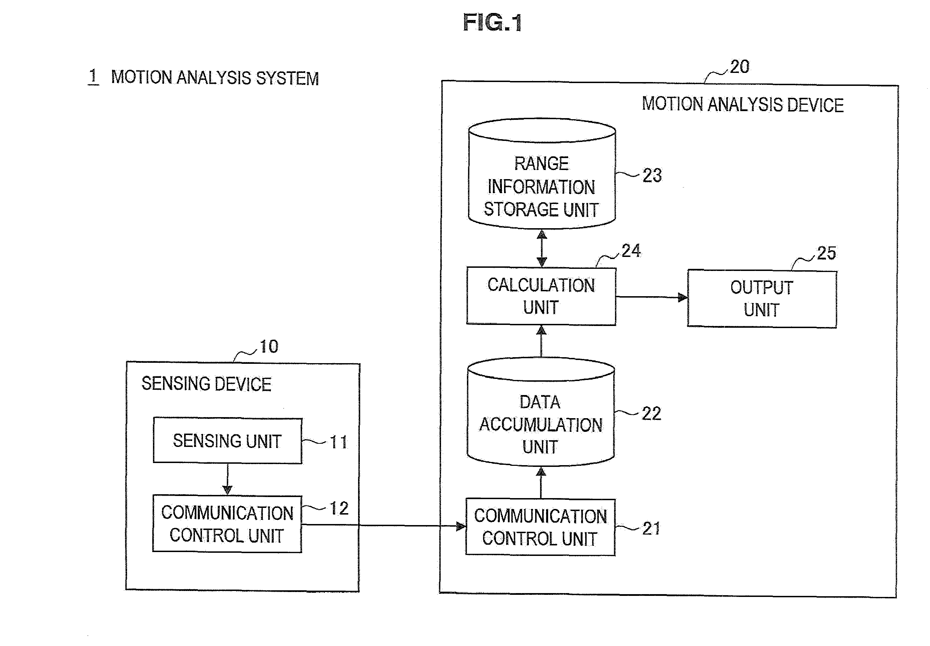

FIG. 1 is a block diagram showing an entire configuration of a motion analysis system 1 of the first embodiment.

The motion analysis system 1 has a sensing device 10 and a motion analysis device 20.

The sensing device 10 is a device for measuring an amount of motion (hereinafter, referred to as "motion amount") of a user who performs a certain motion (hereinafter, referred to as "moving user") and includes a sensing unit 11 for measuring a motion amount and a communication control unit 12 for transmitting measured results to the motion analysis device 20. Here, in the first embodiment, the motion to be analyzed in the motion analysis system 1 is motion of a moving user who is a human being; however, the sensing device 10 may be attached to other objects including an animal or an object (a vehicle such as a car, a machine tool, and the like) other than a human being to analyze motion of those objects. The subject to be analyzed in the motion analysis system 1 is not limited. Further, in the motion analysis system 1, motion to be analyzed is motion which is repeated at a certain amount such as (motion using) a weight training equipment or walking and the type of motion is not limited.

The sensing device 10 is preferably attached to a body part which widely moves such as an upper part of an arm, a wrist or a lumbar area according to the motion category of the moving user; however, the part to which the sensing device 10 is attached is not specifically limited. Further, the sensing device 10 may be fixed to the moving user with a tape or a belt or held in a pocket by the moving user. The method for attaching to the moving user is not limited. Then, when the moving user performs certain motion with the sensing device 10 being attached to the moving user, the sensing device 10 itself moves according to the motion of the moving user, so that the motion amount of the sensing device 10 itself can be recognized as the motion amount of the moving user. In the following description, the sensing device 10 measures an acceleration of the sensing device 10 itself as a motion amount; however, the other physical amounts such as a travel distance or a travel speed per hour may be used.

The sensing unit 11 has functions for measuring a motion amount of the moving user and transmitting the measured result (hereinafter, referred to as "motion data") to the motion analysis device 20 via the communication control unit 12. It is assumed that the sensing unit 11 measures acceleration corresponding to motion of the moving user at every certain cycle (hereinafter, referred to as "sampling cycle") using a known acceleration sensor or the like. Here, the sensing unit 11 may measure acceleration regardless of the sampling cycle and the motion analysis device 20 may extract motion data on a time series and form time series data (hereinafter, referred to as "time series motion data"). In the following description, the sensing unit 11 will be described as a unit that uses a triaxial acceleration sensor; however, a sensor that can observe temporal changes in motion such as an angular speed sensor, a myoelectric potential sensor, a inclination sensor, a Doppler radar, a motion capture from moving images or the like, or a uniaxial or biaxial acceleration sensor may be used. In addition, sensors may be attached to two or more body parts to analyze by integrating values of the respective sensors or select characteristic values.

The communication control unit 12 has a function as an interface for communicating with the motion analysis device 20 and transmits motion data given from the sensing unit 11 to the motion analysis device 20.

The motion analysis device 20 is a device for obtaining motion data from the sensing device 10 and analyzing the motion data to analyze motion performed by the moving user, and includes a communication control unit 21, a data accumulation unit 22, a range information storage unit 23, a calculating unit 24 and an output unit 25.

The motion analysis device 20 is composed by installing a motion analysis program (including fixed data) to an information processing device such as a personal computer (which is not limited to a single device and may be a distributed processing system including two or more devices) and its functions can be illustrated as FIG. 1.

The communication control unit 21 has a function as an interface for communicating with the sensing device 10 and provides motion data obtained from the sensing device 10 to the data accumulation unit 22. Here, the communication between the sensing device 10 and the motion analysis device 20 may be performed by wire or radio and the method is not limited. Further, the transfer of the motion data from the sensing device 10 to the motion analysis device 20 is not necessarily performed on-line and may be performed by off-line by using a CD-ROM, a hard disc or the like. The method of transfer is not limited. Further, in FIG. 1, the motion data is accumulated in the data accumulation unit 22 of the motion analysis device 20; however, the motion data may be transferred to the motion analysis device 20 on-line or off-line after the sensing device 10 accumulates the motion data with another accumulation section.

The data accumulation unit 22 is section for maintaining time series motion data based on motion data by accumulating and storing motion data obtained from the sensing device 10. The data accumulation unit 22 may store motion data by associating with the time obtained from the sensing device 10 or the time measured by the sensing device 10 or may store motion data in chronological order.

The range information storage unit 23 is storage section for storing information related to motion to be analyzed in the motion analysis device 20 and, more specifically, the range information storage unit 23 categorizes motion to be analyzed and holds minimum values and maximum values of the pace of the motion categories.

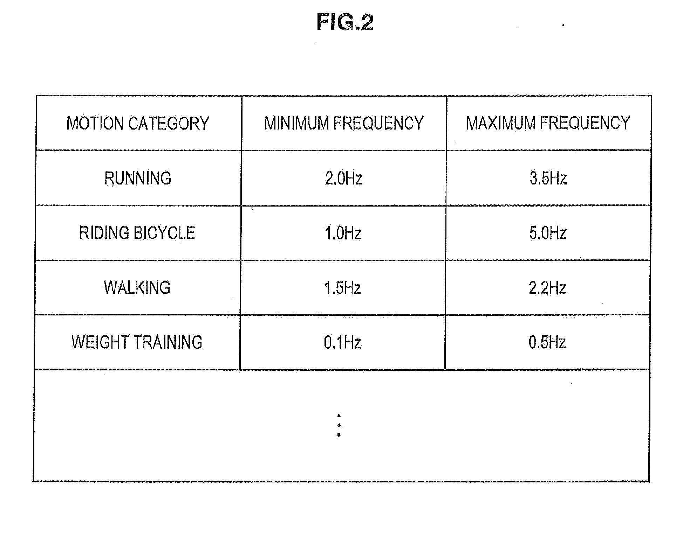

FIG. 2 is an explanatory view showing an example of information stored in the range information storage unit 23.

It is assumed that, in the range information storage unit 23, minimum values and maximum values of paces of the respective motion categories are held in advance as shown in FIG. 2. As a method for calculating the minimum values and maximum values of the paces, for example, the motion of a motion category to be analyzed is repeated with the least speed and with the most speed for a certain period of time and inverse numbers of the numbers of hourly motion may be obtained by eye measurement and registered in advance. The categories shown in FIG. 2 are examples and other motion category may be given or the categories may be more specified or made hierarchically such that the "walking" is divided into "fast walking" and "slow walking." The method of expression is not limited.

In FIG. 2, for example, the pace of "running" is in a range from 2.0 to 3.5 Hz and the pace of "weight training equipment" is in a range from 0.1 to 0.5 Hz.

Here, in FIG. 2, in the respective motion categories, the ranges are shown by the minimum values and maximum values of the paces; however, the registration of the maximum values may be omitted by registering only the minimum values as lower limits of the ranges.

The calculating unit 24 analyzes time series motion data stored in the data accumulation unit 22 by using the data stored in the range information storage unit 23 and gives the analysis result to the output unit 25 to output.

The following description will be made based on an assumption that, when the moving user wears the sensing device 10 and performs motion using a weight training equipment, for example, the calculating unit 24 analyzes time series data stored in the data accumulation unit 22 and analyzes in which level of pace the moving user performs the motion using the weight training equipment (hereinafter, referred to as "motion pace") by using the pace in the range of "0.1 to 0.5 Hz" of "weight training equipment" which are stored in the range information storage unit 23.

The time series motion data to be analyzed by the calculating unit 24 may be motion data sent from the sensing device 10 in real time or the calculating unit 24 may analyze in non-real time by using time series motion data previously accumulated in the data accumulation unit 22.

When the calculating unit 24 analyzes the motion data sent from the sensing device 10 in real time for example, motion data given from the sensing device 10 in an immediate certain period of time may be analyzed. As described above, the range of motion data to be analyzed by the calculating unit 24 or timing of analyzing are not limited.

Further, the motion category to be analyzed by the calculating unit 24 may be set in advance by a user or the like who operate the motion analysis device 20 or the sensing device 10 may include an input unit such as a button so that the moving user can input information related to the motion category that the moving user is going to perform before starting the motion. As described above, the method for specifying motion categories to be analyzed by the calculating unit 24 is not limited.

Here, the details of the motion data analysis by the calculating unit 24 will be described in detail in the following operation description.

The output unit 25 is output section for outputting a result of processing by the motion analysis system 1 and outputs a result of processing by the calculating unit 24 in FIG. 1. The output unit 25 may output to a display device such as a display, store to a storage device such as a disk device or output to a printing device such as a printer. The output method is not limited.

(A-2) Operation of first embodiment

Next, an operation (motion analysis method of the embodiment) of the motion analysis system 1 according to the first embodiment, which has the above structure, will be described.

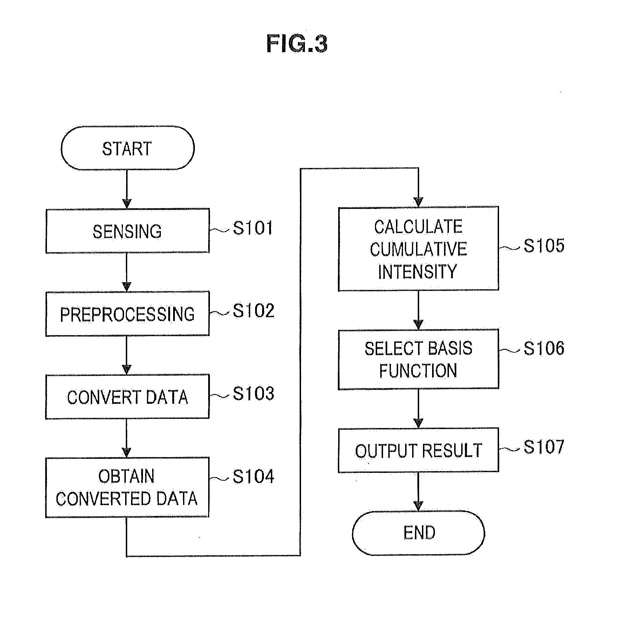

FIG. 3 is a flowchart showing an operation related to an analysis of motion data in the motion analysis device 20.

Here, it is assumed that, in the range information storage unit 23, the content shown in FIG. 2 is held.

Further, in this example, it is assumed that the moving user performs weight training in which his or her arms are trained, and, furthermore, the sensing device 10 is attached to an upper part of an arm of the moving user. Further, it is described under an assumption that the motion category to be analyzed by the calculating unit 24 is set to weight training in advance by the user who operates the motion analysis device 20, for example.

Firstly, when the moving user wears the sensing device 10 and performs motion using a weight training equipment, the motion data is sent from the sensing device 10 to the motion analysis device 20 and accumulated in the data accumulation unit 22 as motion time series data (S101).

Next, as preprocessing prior to analyzing, the calculating unit 24 clips waveform of motion data (acceleration amount time series data) accumulated in the data accumulation unit 22 (S102). Here, when it is already known that the time series motion data accumulated in the data accumulation unit 22 is composed of only motion data related to the motion category to be analyzed (weight training in this example), the process in step S102 may be omitted.

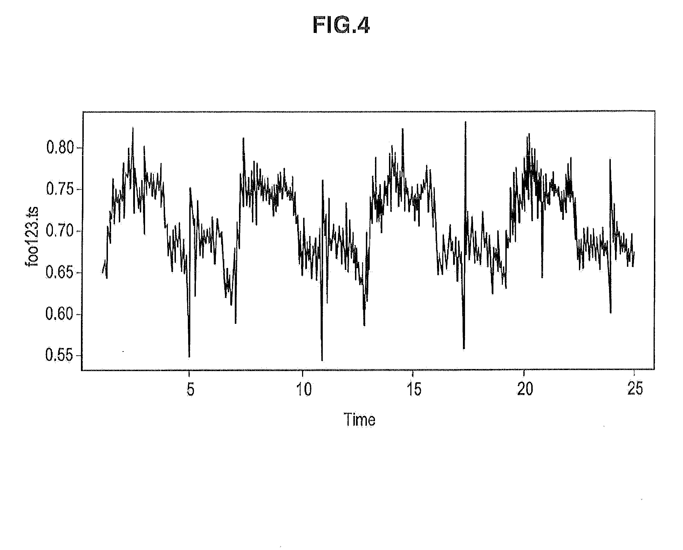

FIG. 4 is a graph showing an example of waveforms clipped by the calculating unit 24 in step S102.

In the following description, the calculating unit 24 will be described on an assumption that the time series motion data shown in FIG. 4 is to be clipped and analyzed. Further, in FIG. 4, the sampling cycle in the time series motion data is 20 milliseconds. Here, the sampling cycle may be changed corresponding to the motion category that is performed by the moving user and is not limited.

In the method for clipping waveforms in step S102, it is preferable to clip waveforms from a beginning to an end of a set of motion when waveforms of the motion category to be analyzed can be clearly distinguished from waveforms of other motion but the waveforms may be clipped at certain time interval when the difference from the waveforms of other motion is not clear. The method for clipping waveforms is not limited. For example, when the timings when the moving user starts and ends motion of a motion category to be analyzed are known in advance, the clipping of waveforms in step S102 may be performed by clipping any selected waveforms within the time while the moving user is performing the motion to be analyzed. Regarding the motion analysis device 20, as a method for obtaining timings when the moving user starts or ends motion of the motion category to be analyzed, for example, those timings may be set by a user who operates the motion analysis device 20 or the sensing device 10 may include an input unit such as a button so that the moving user can input timings when the moving user starts and ends the motion. The method is not limited. Further, in the above described step S102, preprocessing to distinguish a difference in waveforms such as a biaxial or triaxial waveform combining or an application of a low-pass filter may be executed at this stage according to need.

Next, the calculating unit 24 performs a data conversion on the waveforms, which are clipped in step S102 and shown in FIG. 4, with a correlation calculation using plural frequencies (S103).

In step S103, more specifically, a wavelet transform using the following equation (1) is performed. In the following equation (1), "ψ" represents a mother wavelet function, "f(t)" represents the waveforms shown in FIG. 4, "a" represents a scale showing a level of an expansion or a contraction of an applied wavelet and "b" represents a travel distance of wavelet in a time axis direction. Here, in the calculating unit 24, types of the mother wavelet function to be used and parameters thereof are not limited. Note that, in this example, in the following equation (1), "a" is in a range from 0 to 1 (seconds) and "b" is in a range from 0 to 30 (seconds).

Further, when it is assumed that "Δ" represents a sampling cycle and "$F_C$" represents a center frequency of mother wavelet, a frequency "$F_a$" corresponding to scale "a" can be calculated based on the following equation (2).

Further, when the following equation (3) is applied, "p" (scalogram) corresponding to the intensity of the wavelet can be calculated. Here, since "p" corresponding to the intensity of the wavelet also corresponds to "spectral intensity" in frequency related to the wavelet, "p" will be referred to as "spectral intensity" in the following description.

$$WT(b, a) = \frac{1}{\sqrt{|a|}} \int_{-\infty}^{\infty} f(t) \overline{\psi\left(\frac{t-b}{a}\right)} dt \qquad \cdots (1)$$

$$F_a = \frac{F_C}{a \cdot \Delta} \qquad \cdots (2)$$

$$p(b, a) = \left| WT(b, a) \right|^2 \qquad \cdots (3)$$

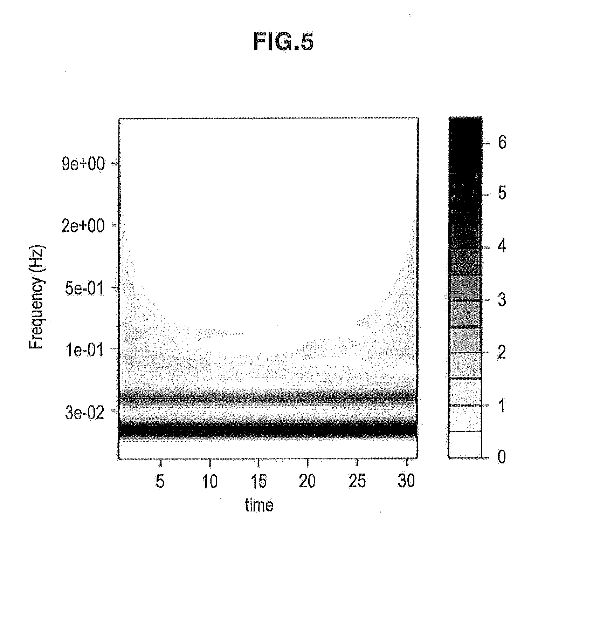

FIG. 5 is a graph showing results of wavelet transform based on the waveforms shown in FIG. 4.

The graph of FIG. 5 is a graph (scalogram) which expresses so that the horizontal axis represents time (t), vertical axis represents frequencies ("$F_a$") and the color tone (density of stippling) represents spectral intensities (p). Here, in FIG. 5, as a darker color tone (higher density of stippling) represents a higher spectral intensity.

When the data conversion in step S 103 ends, the calculating unit 24 further clips data from the converted data and the clipped data is obtained (S104).

Focusing on the right and left ends of the graph shown in FIG. 5, an area having a spectral intensity relatively higher than surrounding area spreads in a U-shape as the frequency becomes low. This is because the applied time length becomes greater when the frequency is lower or, in other words, when the level of the expansion or contraction of mother wavelet during the wavelet transform is greater, the wider region (time) is to be converted including the region (time) where there is no motion data. When the motion data to be analyzed has sufficient length of time, the above influence is hardly seen; however, it may not be ignored when data in a short period of time such as motion with a weight training equipment is analyzed.

Thus, in step S104, a process is executed to clip data in the time axis direction only in a range where the motion pace of weight training is assured at a minimum level. More specifically, in step S104, the calculating unit 24 truncates information in both ends using the minimum value of the pace of each category stored in the range information storage unit 23. Here, since the process in step S104 is a step for improving the accuracy of analysis results by the calculating unit 24, this step may be omitted according to the level of accuracy that is required.

Next, details of the process of clipping the converted data in step S104 will be described.

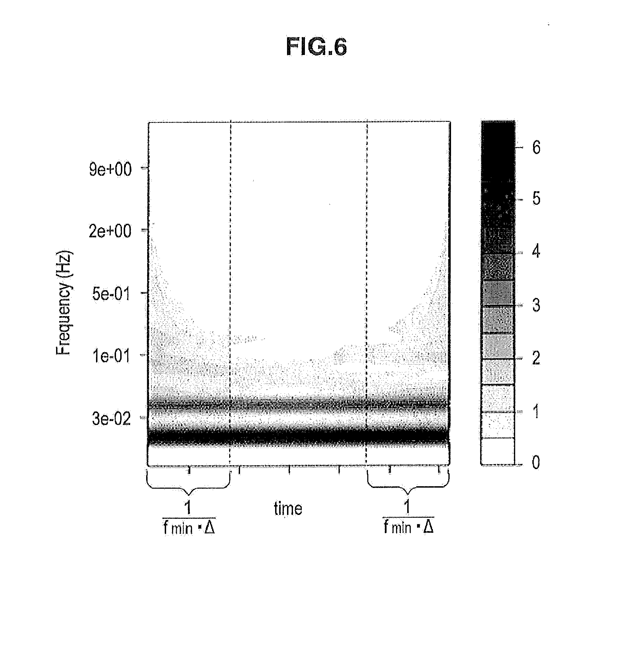

FIG. 6 is an explanatory view showing clipping of converted data performed in step S104.

When it is assumed that the sampling cycle in the sensing device 10 is "$\Delta$" (= 20 milliseconds), the center frequency of the mother wavelet is "$F_C$", the minimum frequency of the motion category to be analyzed is "$f_{min}$", and the scale corresponding to thereof is "$a_{min}$", an analysis of the above frequency range is assured as long as the length of the time given in the following equation (4) is maintained; however, as indicated by the following equation (4), the "time length to be truncated" can be obtained as "$1/(f_{min} \cdot \Delta)$" as a result. Here, in this example, in the calculating unit 24, it is set in advance that the motion category to be analyzed is weight training as described above, "$f_{min}$" is relevant to "0.1 Hz" that is the minimum frequency of weight training as shown in FIG. 2.

Thus, in step S104, as shown in FIG. 6, data is obtained by truncating the data in both right and left ends (in a time axis direction) by the amount of "$1/(f_{min} \cdot \Delta)$" in the graph shown in FIG. 5 as an effective region.

$$\text{time length to be truncated} = a_{min} \cdot \frac{1}{Fc} = \frac{Fc}{f_{min} \cdot \Delta} \cdot \frac{1}{Fc} = \frac{1}{f_{min} \cdot \Delta} \qquad (4)$$

When the data clipping in step S 104 ends, the calculating unit 24 calculates a cumulative value of spectral intensities in each frequency in the effective regions clipped in S104 (S105).

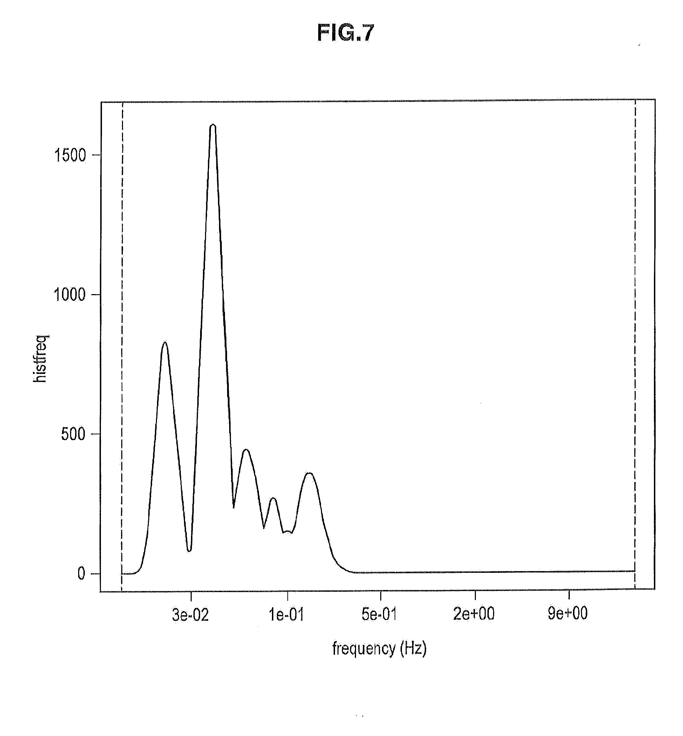

FIG. 7 is a graph (scalogram) showing cumulative values of spectral intensity calculated by the calculating unit 24 in step S104.

In FIG. 7, the vertical axis indicates cumulative values of spectral intensities and the horizontal axis indicates frequencies. In FIG. 7, the peak of the spectral intensity which are expressed using torn in FIG. 6 are shown in the vertical axis of the graph, and the overall level of frequency component within the time period of motion to be analyzed can be observed. Here, a method for evaluating the level of frequency component based on the cumulative value of the spectral intensities has been described; however, a method for calculating a maximum value of spectral intensities in each frequency in the effective regions may be used.

In step S105, in the calculating unit 24, after the spectral intensity cumulative value shown in FIG. 7 is calculated, a basis function selection of frequency related to the motion pace of the moving user, that is an analysis of the motion pace of the moving user, is performed (S106). As shown in FIG. 7, plural local maximum values of spectral intensity are found in low-frequency components.

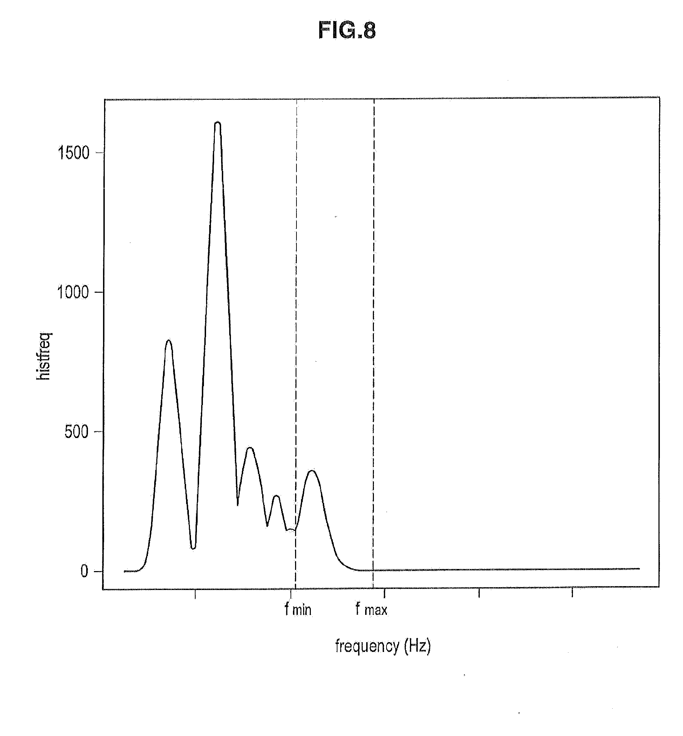

FIG. 8 is an explanatory view showing a manner how the motion pace of the moving user is analyzed in calculating unit 24.

Referring back to the range information storage unit of FIG. 4 to extract frequency corresponding to the pace of motion, the minimum value "$f_{min}$" and maximum value "$f_{max}$" of the pace in each motion category are used. In other words, as shown in FIG. 8, local maximum value is searched focusing on a range from equal to or greater than $f_{min}$ and equal to or lower than "$f_{max}$" in the graph of the spectral intensity cumulative values. Here, as described above, in the calculating unit 24, since it is set in advance that the motion category to be analyzed is weight training, "$f_{min}$" is relevant to 0.1 Hz and "$f_{max}$" is relevant to 0.5 Hz based on the content of FIG. 2.

In other words, regarding the motion using a weight training equipment, since it is assumed that the minimum value of the pace is "$f_{min}$" (0.1 Hz) and the maximum value of the pace is "$f_{max}$" (0.5 Hz), the component related to the motion pace of the moving user is included in the range from 0.1 to 0.5 Hz in the spectral intensity cumulative values and the frequency having the local maximum spectral intensity cumulative value thereof can be estimated as the motion pace performed by the moving user. Note that, in the following description, the spectral intensity in the motion pace or the spectral intensity cumulative value will be referred to as "motion pace spectrum."

Then, the frequency having the local maximum value extracted in step S 106 is output as the pace of the motion (S107).

(A-3) Effects of first embodiment

According to the first embodiment, the following effect can be achieved.

Referring to the waveforms related to the motion using a weight training equipment in FIG. 4, it is obvious that to obtain a proper peak value or a zero-crossing is difficult without preprocessing, accordingly, it is difficult to obtain the paces of various types of motion in a collective manner in the technologies described in conventional Patent Documents 1 to 7. On the other hand, as the motion analysis system 1 described in the first embodiment, the analysis of waveforms using a wavelet transform basically is a method for expressing input waveforms given by adding expanded, contracted or translated mother wavelets, so that the width of mother wavelet changes corresponding to frequencies and the frequency resolution is much improved than a spectral analysis simply using Fourier transform.

In the calculating unit 24, since the waveforms related to time series motion data is analyzed using a multiscale analysis such as a wavelet transform, relation between the time length applied for transforming and the frequency of the subject to be analyzed is in inverse proportion and the lower frequencies can be analyzed with a higher resolution. More specifically, in the calculating unit 24, regarding the mother wavelet (see the above equation (1)) which is applied to the waveforms indicated by the time series motion data, a parameter (scale a) corresponding to the frequency "$F_a$" to be analyzed is applied based on the above equation (2), so that lower frequencies realize a multiscale analysis with a higher resolution. Thus, according to the motion analysis system 1, regarding motion of various paces including a low-frequency pace such as motion using a weight training equipment, a high-accuracy analysis of motion pace or the like can be performed by the same analysis method without processing related to shape of waveforms. Then, in the motion analysis system 1, regarding motion of various paces, a high-accuracy analysis can be performed using the same analysis method without preprocessing, throughputs or resources required for an analysis can be reduced.

(B) Second embodiment

A second embodiment of a motion analysis device, program and method, and a motion analysis system according to the present invention will be described in detail with reference to the drawings.

(B-1) Configuration of second embodiment

In the first embodiment, a method for detecting a pace of motion based on motion data has been described; however, in the second embodiment, a method for analyzing a physical amount related to a load of motion of a moving user will be described. The physical amount related to a load of motion of a moving user is, for example, a load amount of a weight training equipment or consumed calories of the moving user in a case where the motion category to be analyzed is weight training.

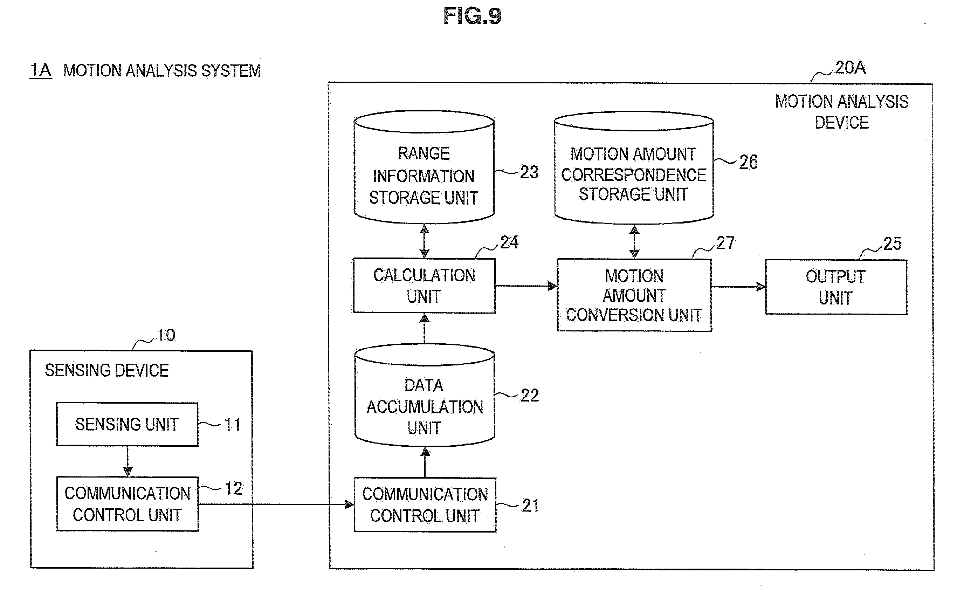

FIG. 9 is a block diagram showing an entire configuration of a motion analysis system 1A according to this embodiment.

The motion analysis system 1A includes a sensing device 10 and a motion analysis device 20A. The sensing device 10 is the same as that of the first embodiment, so detail description will be omitted.

The motion analysis device 20A basically has the same configuration as the motion analysis device 20 of the first embodiment except that the motion analysis device 20A has a motion amount conversion unit 26 and a motion amount correspondence storage unit 27.

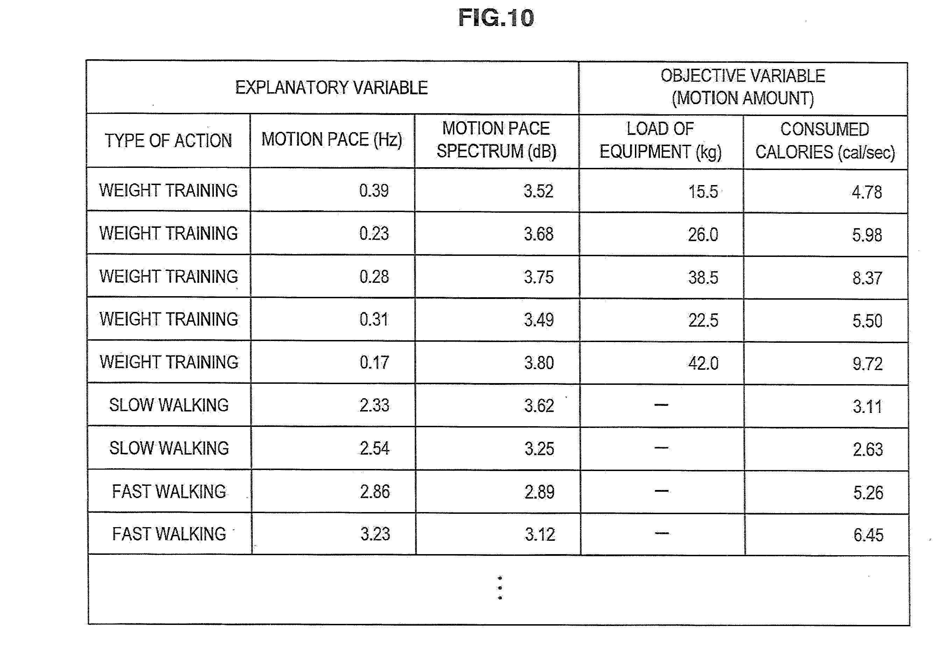

FIG. 10 is an explanatory view showing an example of information stored in the motion amount correspondence storage unit 27.

In the motion amount correspondence storage unit 27, as shown in FIG. 10, information of explanatory variables corresponding to the types of motion categories (motion pace, motion pace spectrum) and objective variables (load of equipment, consumed calories) are registered. Here, in FIG. 10, at least one piece of information may be registered respectively for the explanatory variable and objective variable. For example, when the motion category is "weight training," the item of the motion pace as an explanatory variable may be omitted or the item of consumed calories as an objective variable may be omitted. Further, in FIG. 10, when the motion category is slow walking or fast walking, the spectral intensity as an explanatory variable may be omitted. Further, in FIG. 10, when the motion category is slow walking or fast walking, there is no idea of load of equipment as an objective variable, so the item remains blank. The information stored in the motion amount correspondence storage unit 27 shown in FIG. 10 may be registered in advance.

For example, in FIG. 10, when the motion category is "weight training equipment," it is assumed that the pace is "0.39 Hz," the spectral intensity cumulative value is "3.52 dB," the load of equipment is "15.5kg" and the consumed calories are "3.78 cal/sec." Here, the motion pace and motion pace spectrum (see FIG. 8) calculated in the calculating unit 24 may be maintained in advance when the moving user performs motion of "weight training" with a load of equipment of "15.5kg" as wearing the sensing device 10, those values may be registered. Regarding the consumed calories, generally, consumed calories of motion of "weight training" with a load of equipment of "15.5kg" may be registered, or the values measured by attaching a known measurement device to the moving user when the spectral intensity cumulative value in the motion pace is obtained may be stored. The method for obtaining consumed calories is not limited.

Here, in FIG. 10, five pieces of information related to the motion category of "weight training" are registered; however, if there are at least two pieces of information, a later described analysis in the motion amount conversion unit 26 can be available while more pieces of information improves the level of accuracy of analysis.

Next, a configuration of a process, in motion amount conversion unit 26, for analyzing a physical amount related to a load of motion.

In the motion amount conversion unit 26, since the motion category to be analyzed is the same as that of the calculating unit 24, the description of the method for setting thereof will be omitted. In the following description, as an example, it is assumed that the motion category to be analyzed in the motion amount conversion unit 26 is "weight training."

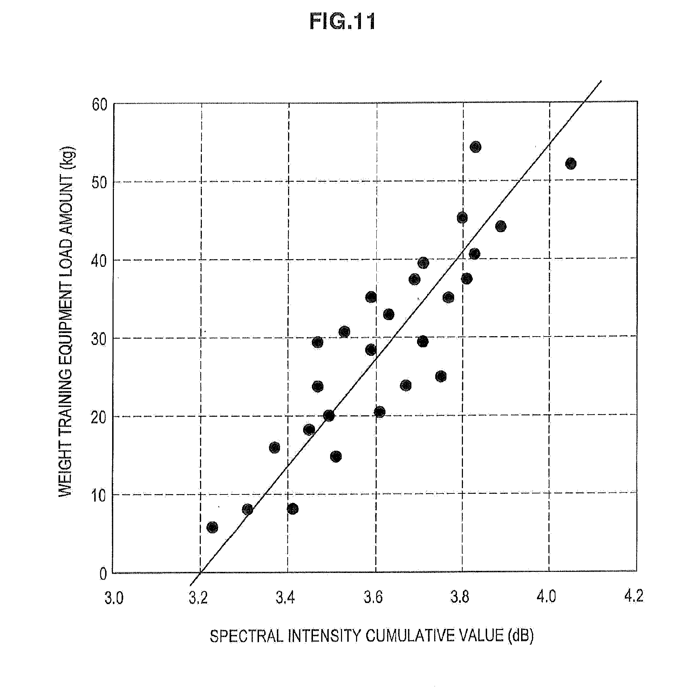

FIG. 11 is an explanatory view showing a relationship between motion pace spectrums and the load amount of the weight training equipment.

FIG. 11 shows of a manner of a linear simple regression analysis in which the motion pace spectrum is set as an explanatory variable and the load amount of the weight training equipment as a physical amount related to load of motion is set as an objective variable.

In the motion amount conversion unit 26, the combination of the explanatory variable and objective variable in analysis is not limited and analysis may be performed based on plural combinations. In this example, the combination shown in FIG. 11 will be described as an example.

FIG. 11 shows a relation between the spectral intensities and the load amounts of the weight training equipment, using data in the motion amount correspondence storage unit 27. Then, in the motion amount conversion unit 26, a regression equation shown as the following equation (5) is obtained based on the relationship shown in FIG. 11. Then, in the motion amount conversion unit 26, an "equipment load" when the moving user performs motion using a weight training equipment can be estimated by applying the following equation (5) to the spectral intensity cumulative value (see FIG. 8) in the motion pace of the moving user, which is estimated in the calculating unit 24 using the newly obtained motion data. In the motion amount conversion unit 26, when a calculation is executed using the consumed calories as a substitute for the objective variable, "consumed calories" when the moving user performs motion causing a weight training equipment can also be estimated.

(load amount of weight training equipment)

$= 66.7 \times$ (spectral intensity cumulative value) $- 213.3$ ...(5)

Here, in the motion amount conversion unit 26, as a method for determining a combination of the explanatory variable and objective variable when analyzing, combinations may be set in the respective motion categories in advance or only one explanatory variable and one objective variable may be registered when information is registered in the motion amount correspondence storage unit 27. The method for determining a combination is not limited. For example, in the case where the motion category is weight training, only motion pace spectrum may be input as an explanatory variable and only load of equipment may be input as an objective variable, or a combination may be set in advance.

Then, in the motion analysis device 20A, the output unit 25 output an estimation result of the motion amount conversion unit 26. Here, the output unit 25 may output the estimation result of the calculating unit 24 with the estimation result of the motion amount conversion unit 26.

(B-2) Operation of second embodiment

Next, an operation (a motion analysis method of the embodiment) of the motion analysis system 1A having the above described configuration according to the second embodiment will be described.

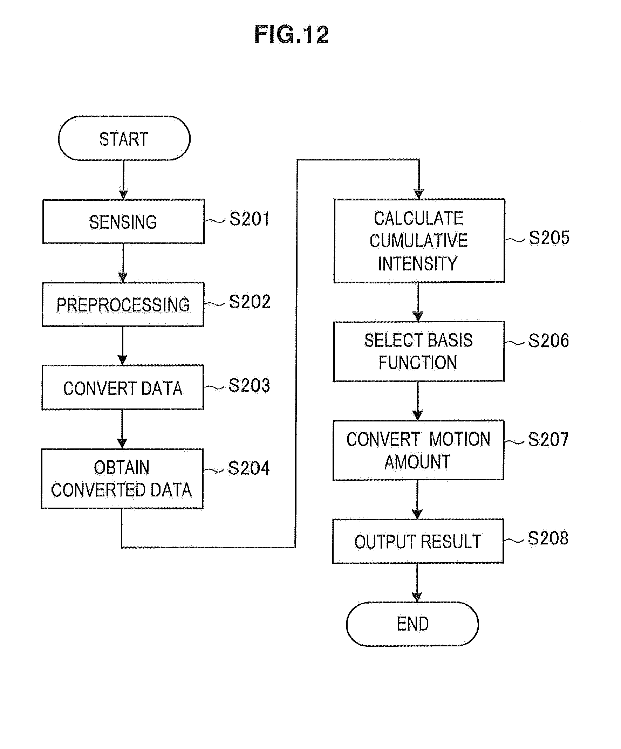

FIG. 12 is a flowchart showing an operation related to a motion data analysis in the motion analysis device 20A.
Here, it is assumed that the range information storage unit 23 holds the content shown in FIG. 2 and the motion amount correspondence storage unit 27 holds the content shown in FIG. 10.
Further, in this example, it is assumed that the moving user performs weight training including motion of his or her arms and the sensing device 10 is attached to an upper part of the moving user's arm. Further, in the calculating unit 24 and motion amount conversion unit 26, weight training is previously set as the motion category to be analyzed by a user who operates the motion analysis device 20. Further, in the motion amount conversion unit 26, it is assumed that the explanatory variable used in the analysis is previously set to a motion pace spectrum and the objective variable is previously set to load of equipment. This is because that the load of equipment is strongly related to the motion pace spectrum in the motion using a weight training equipment.
Here, in FIG. 12, the processes in steps S201 to S205 are the same as the processes in steps S 101 to S 105 in FIG. 3, so those descriptions will be omitted.
Then, in processes in steps S201 to S205, in the calculating unit 24, the process up to searching a local maximum value (motion pace spectrum) among spectral intensity cumulative values focusing on the range from $f_{min}$ to $f_{max}$ in the graph of FIG. 8 showing spectral intensity cumulative values in each frequency are the same as the process in step S106 of FIG. 3, and, in the second embodiment, the motion amount conversion unit 26 obtains the motion pace spectrum searched by the calculating unit 24 (S206).
In step S206, the motion amount conversion unit 26 may obtain only the motion pace spectrum corresponding to the explanatory variable used in the analysis. In this example, as described above, since the explanatory variable used in the analysis is only motion pace spectrum, the motion amount conversion unit 26 obtains only the motion pace spectrum in S206; however, the value of the motion pace may be obtained in the case where the motion pace is also set as the objective variable.
Then, when the motion amount conversion unit 26 obtains the motion pace spectrum in step S206, the motion amount conversion unit 26 analyzes the "equipment load" while the moving user performs motion using a weight training equipment based on the obtained motion pace spectrum and information of the motion amount correspondence storage unit 27 and provides the result to the output unit 25 (S207).
Then, the analysis result by the motion amount conversion unit 26 in step S207 is output by the output unit 25 (S107).

(B-3) Effects of second embodiment

According to the second embodiment, the following effects are achieved in addition to the effects of the first embodiment.
In the motion analysis system 1A, a motion amount of motion to be analyzed such as a load amount of a weight training equipment or consumed calories can be estimated in motions slower than walking. Further, as described in the first embodiment, the pace of motion and a physical amount related to a load of motion can be detected at the same time and this helps to recognize a condition of a person in a health condition management or rehabilitation more specifically.

(C) Third embodiment

A third embodiment of a motion analysis device, a program, a method and a motion analysis system according to the present invention will be described in detail with reference to the drawings.

(C-1) Configuration of third embodiment

In the first embodiment, a method for detecting a pace of motion based on motion data has been described. In the third embodiment, a detection of an abnormal action during motion will be described. Here, an abnormal action represents an action which speed is faster than subject motion at a certain time period among motion that repeats a certain action as a subject to detect a pace or a motion amount. For example, when lifting a handle of a weight training equipment to which a load (weight) is being applied, dropping the handle suddenly or releasing the handle by the moving user who cannot hold the load applied to the handle are included to abnormal actions.

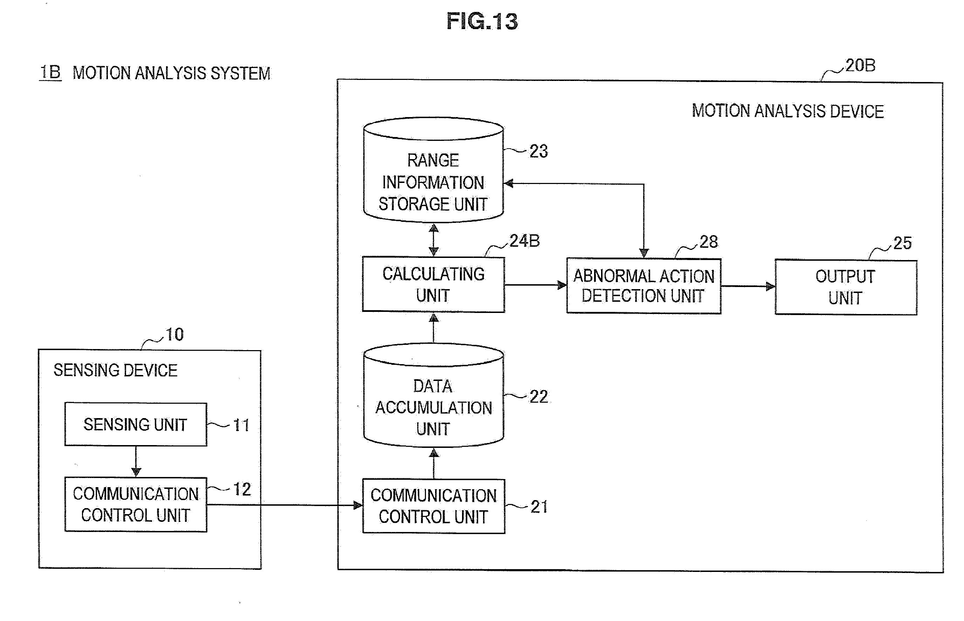

FIG. 13 is a block diagram showing an entire configuration of a motion analysis system 1B of the embodiment.

The motion analysis system 1B includes a sensing device 10 and a motion analysis device 20B and, since the sensing device 10 is the same as that of the first embodiment, detail description thereof will be omitted.

The motion analysis device 20B has a calculating unit 24B as a substitute for the calculating unit 24 and further includes an abnormal action detection unit 28, compared to the motion analysis device 20 of the first embodiment and other configurations are the same, so only the difference from the first embodiment will be described below.

The process of the calculating unit 24B to obtain the content of graph (scalogram) shown in FIG. 5 based on the motion data accumulated in the data accumulation unit 22 is the same as the process of the calculating unit 24 of the first embodiment.

Then, the calculating unit 24 of the first embodiment obtains spectral intensity cumulative values in each frequency in the whole region (time) of the effective region based on the scalogram shown in FIGS. 5 and 6, and differently from the first embodiment, the calculating unit 24B of the third embodiment divides the effective region by time windows of a certain zone length ("2 seconds," for example) and calculates spectral intensity cumulative values in each frequency in each zone. Here, similarly to the first embodiment, the motion analysis device 20B may employ a method for calculating the maximum value of spectral intensity in each frequency in effective region, without using the spectral intensity cumulative value.

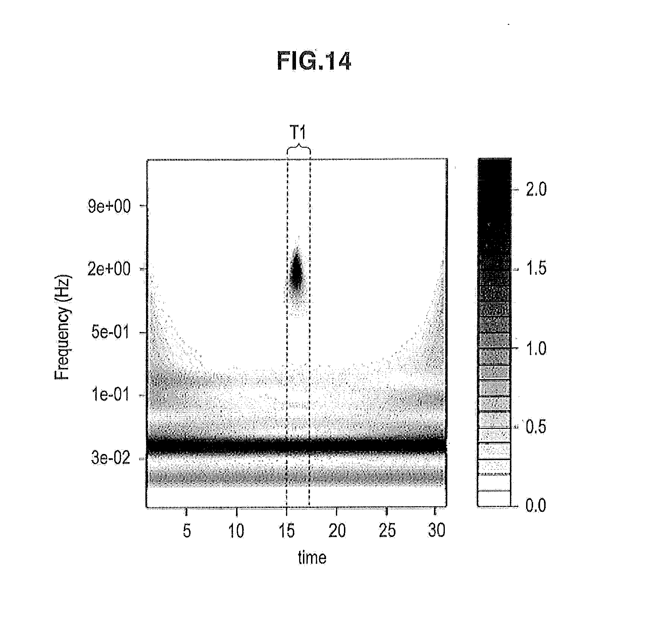

FIG. 14 is a graph (scalogram) created based on motion data when an abnormal action occurs while the moving user wares the sensing device 10 and performs motion using a weight training equipment. FIG. 14 shows a manner an abnormal action occurs in a zone T1 on the time axis.

The abnormal action detection unit 28 determines whether an abnormal action is included in the motion data accumulated in the data accumulation unit 22 based on the calculation result of the calculating unit 24B and the information stored in the range information storage unit 23.

Hereinafter, a method for detecting an abnormal action in the abnormal action detection unit 28 will be described.

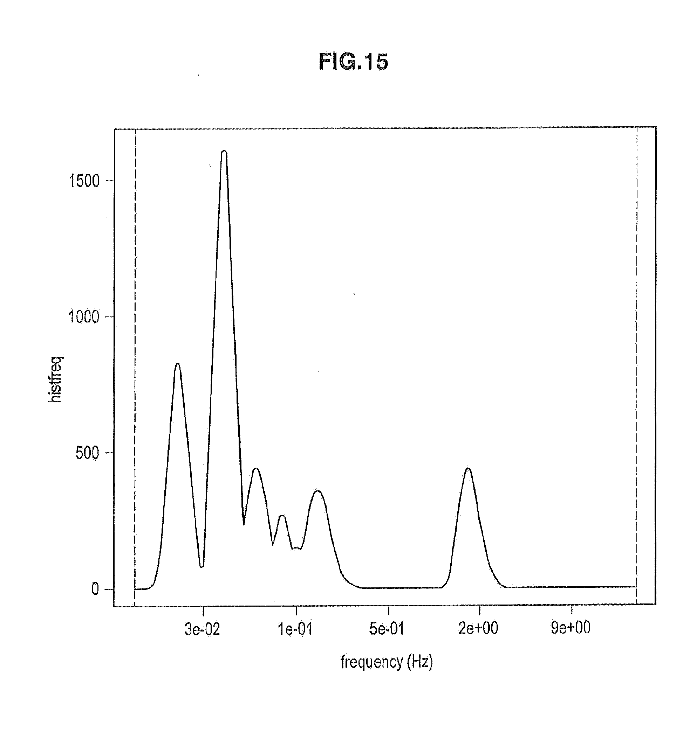

FIG. 15 is a graph in a case where spectral intensity cumulative values in each frequency in a time window including a zone T1 in which an abnormal action occurs are calculated.

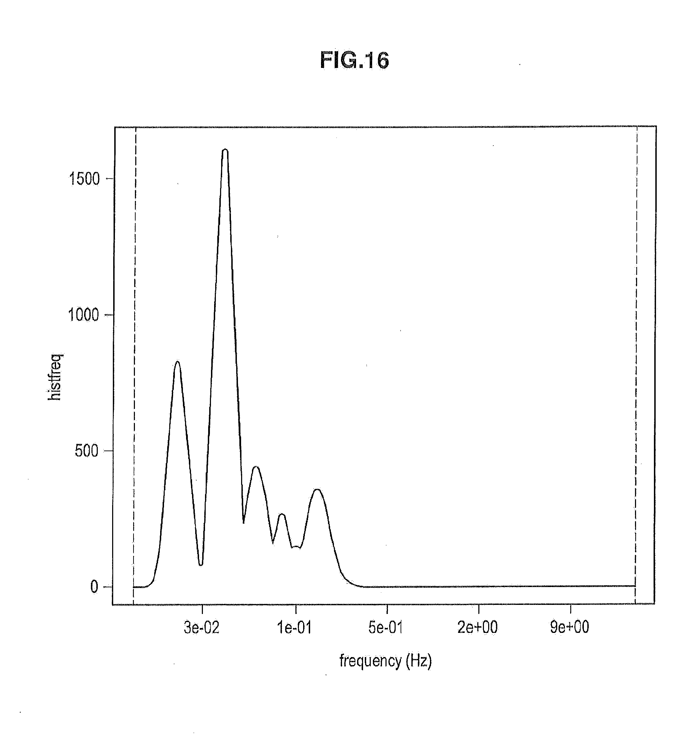

FIG. 16 is a graph in a case where spectral intensity cumulative value in each frequency in a time window which does not include the zone T1 in which the abnormal action occurs are calculated.

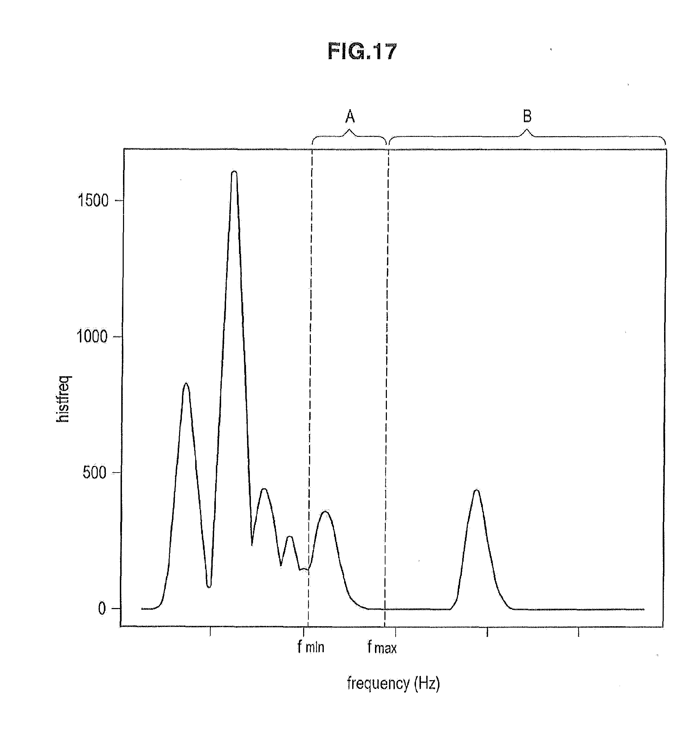

FIG. 17 is an explanatory view showing a manner of detecting whether there is an abnormal action in the graph related to the time window including the zone T1 in which the abnormal action shown in FIG. 15 occurs.

As described above, the calculating unit 24B divides the effective region by time windows of a certain zone length and calculates spectral intensity cumulative values in each frequency in the zones. In FIG. 15, there is a peak in a region of relatively high frequency, which is not in FIG. 16.

The abnormal action detection unit 28 determines whether there is an abnormal action based on whether a peak in a region of relatively high frequency as shown in FIG. 15.

Hereinafter, an example of a concrete method for determining whether there is an abnormal action will be described. For example, referring to the range information storage unit 23, the abnormal action detection unit 28 obtains a minimum value "$f_{min}$" and a maximum value "$f_{max}$" of the paces of each category of motion from which the pace and motion amount are detected, searches a local maximum value in a region from "$f_{min}$" to "$f_{max}$" (the region A of FIG. 17), and obtains a motion pace spectrum. Next, the range information storage unit 23 searches a local maximum value in a region greater than "$f_{max}$" (the region B of FIG. 17) and obtains the spectral intensity (hereinafter, referred to as "abnormal action spectrum"). Then, the range information storage unit 23 compares the two spectral intensity. When the abnormal action spectrum is greater than the motion pace spectrum, it may be determined that there is an abnormal action and when the abnormal action spectrum is not greater than the motion pace spectrum, it may be determined that there is not an abnormal action. Here, in the abnormal action detection by the abnormal action detection unit 28, a proper coefficient may be multiplied to one of the spectral intensities to compare in order to adjust a threshold value for the abnormal action determination.

As described above, the outputting method in output unit 25 is not limited, and the output of the analysis and the analysis result by the motion analysis system 1B may be applicable in real time or in non real time. For example, when the analysis and analysis result are output in real time, an analysis result including an abnormal action may be notified (a lamp, a buzzer, an e-mail and the like; the method of notification is not limited) to a staff (instructor) in a gym and the like.

(C-2) Operation of third embodiment

Next, an operation (a motion analysis method of the embodiment) of the motion analysis system 1B of the third embodiment having the above configuration will be described.

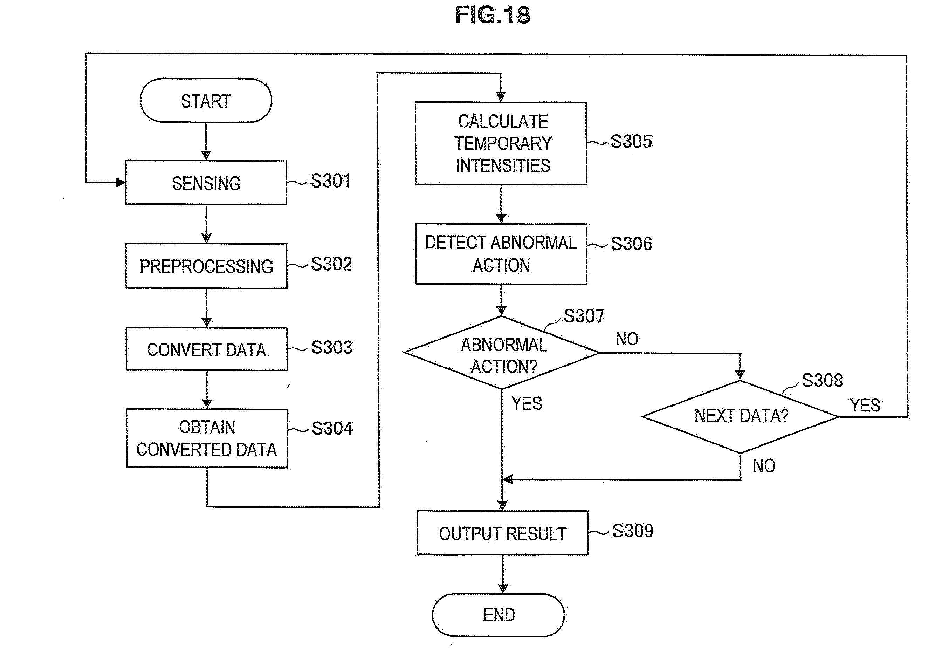

FIG. 18 is a flowchart showing an operation related to an analysis of motion data in the motion analysis device 20B.

Here, it is assumed that the content shown in FIG. 2 is held in a range information storage unit 23.

Further, in this example, it is assumed that the moving user performs motion using a weight training equipment to move his or her arms and the sensing device 10 is attached to an upper part of the moving user's arm. Further, in the calculating unit 24 and abnormal action detection unit 28, it is assumed that the motion category to be analyzed is set to weight training equipment in advance by a user who operates the motion analysis device 20 for example.

Here, in FIG. 18, the processes in steps S301 to S304 are the same as those in step S101 to S104 of FIG. 3, and the description thereof will be omitted.

In the first embodiment, spectral intensity cumulative values in each frequency in the "entire" effective region in step S105 (calculating cumulative intensity); however, in the third embodiment, the effective region is divided into time windows of a certain zone length and spectral intensity cumulative values in each frequency in those zones are calculated (S305) as described above.

Based on the calculation result by the calculating unit 24B and the information stored in the range information storage unit 23, it is determined that there is an abnormal action in the motion data accumulated in the data accumulation unit 22 (S306). More specifically, the abnormal action detection unit 28 determines whether an abnormal action is included in every time window based on the calculation result in step S305. In S305, the abnormal action detection unit 28 may determine as a final determination result that there is an abnormal action when it is determined that there is an abnormal action in at least one time window, or may determine as a final determination result that there is an abnormal action only when abnormal actions are included in the predetermined threshold number or more of time windows.

Then, when it is determined that there is an abnormal action in step S306, the determination result is output as a result display, when an abnormal action is not detected in step S306, the process returns to step S301 and continues as long as data from sensing device 10 are accumulated in the data accumulation unit 22 (S307 to S309).

Here, in steps S307 to S309, it is determined whether to output determination result according to the determination result in step S306; however, the determination result in step S306 may be uniformly output when there is no abnormal action.

(C-3) Effects of third embodiment

According to the third embodiment, the following effects are achieved in addition to the effects of the first embodiment.

The motion analysis system 1B can detect an abnormal action which is faster than subject motion in a certain time period in motion repeating a certain action. Here the present embodiment is applicable to real-time or non-real-time processes and the real-time process can be used in a gym or the like to detect an dangerous action and notify an instructor of the action and the non-real-time process can be used in a field of motion analysis to find a difference between proper motion and improper motion.

Further, when the motion analysis device 20B notifies a staff (instructor) of an analysis result in real time, the staff (instructor) is able to aware of the moving user who had an abnormal action so that the safety of the moving user can be assured. Especially, an abnormal action may harm the safety of the moving user in motion using a weight training equipment or the like that applies a heavy load to the moving user, the detection of an abnormal action is important. Further, when the motion analysis device 20B outputs the analysis result in non real time, the results are used to find a difference between proper motion and improper motion in a field of motion analysis.

(D) Other embodiments

It should be understood by those skilled in the art that various modifications, combinations, sub-combinations and alterations may occur depending on design requirements and other factors insofar as they are within the scope of the appended claims or the equivalents thereof.

(D-1) In the above embodiments, examples in which a wavelet transform is used in a function for a data conversion in the calculating unit have been described; however, it is not limited to a wavelet transform as long as a correlation calculation can be executed using plural frequencies, and Fourier transform, Hadamard transform, cosine transform or Hilbert transform may be employed. Here, the method using Hilbert transform is described in "The latest wavelet practical lesson - introduction and application - from the basic signal processing to the latest theory" by Hiroshi TODA, Zhong ZHANG and Hiroaki KAWABATA, SoftBank Creative Corp., September, 2005.

(D-2) In the first embodiment, to explain based on the wavelet transform, the range information storage unit 23 show in FIG. 2 maintains only a range of frequencies in motion categories and calculates the time length to be truncated is uniquely based on the frequency using the above equation (4). However, when a method other than the wavelet transform is employed, the time length to be truncated is previously set to the range information storage unit 23 to determine an effective region.

(D-3) In the above embodiments, a method for extracting the pace of sensor data obtained by referring to the region of frequencies of motion stored in the range information storage unit 23 has been described. However, in contrast, a method of behavior recognition may be employed so that, based on the waveforms of the time series motion data accumulated in data accumulation unit 22, a motion category to which the motion belongs to can be estimated by comparing with the frequencies of motion stored in the range information storage unit 23.

(D-4) The method for estimating the motion amount in the second embodiment is not limited to the above described linear simple regression analysis. The method may be expanded to a nonlinear regression analysis, a multiple regression analysis in which a subject type of action or a pace are added to an explanatory variable may be employed. There are further effective estimations in which a discriminant analysis such as a multiclass support vector machine, a neural network and a k-nearest neighbor method and the discrete values are obtained as a result. When a subject type of action is used as an explanatory variable, the type of action may be estimated based on the acceleration waveforms.

(D-5) In the second embodiment, the physical amount to be analyzed related to the load caused by motion by the moving user may include an angle of a treadmill, a carbon dioxide levels in exhaled breaths, a lactate level in addition to the above mentioned load amount of weight training or consumed calories or an evaluation index of a motion amount in a correlation with the spectral intensity may be newly defined.

(D-6) In the third embodiment, as shown in FIG. 17, only local maximum value of spectral intensity cumulative values existing in the region B (the region greater than "$f_{max}$") is used to determine whether there is an abnormal action or not; however, the local maximum value of spectral intensity cumulative values in a frequency band which is higher than the motion pace by a certain level in the region A (the region from "$f_{min}$" to "$f_{max}$") may be used to determine whether there is an abnormal action or not.

For example, even in the region A (the region from "$f_{min}$" to "$f_{max}$"), a local maximum value of spectral intensity cumulative values in a frequency band which is 1.5 times higher than the motion pace (for example, a band of 1.5 Hz or higher when the motion pace is 1 Hz) may be used to determine whether there is an abnormal action or not.

The present application contains subject matter related to that disclosed in Japanese Priority Patent Application JP 2009-115720 filed in the Japan Patent Office on May/12/2009, the entire content of which is hereby incorporated by reference.

## Claims

1. A motion analysis device constituting a motion analysis system that includes a sensor device for measuring a motion amount related to motion of an object which repeats a certain motion and the motion analysis device for analyzing the certain motion of the object based on the measured result of the sensor device, the motion analysis device comprising:

   time series data storing section configured to store time series data of the motion amount measured by the sensor device;
   waveform analyzing section configured to analyze a spectral intensity in each frequency by applying a certain function to a waveform shown by the time series data and analyze by applying different parameters for each frequency to the function; and
   motion analysis section configured to compare, for different frequencies, the spectral intensity analysis result in each frequency analyzed by the waveform analyzing section and analyze a certain physical amount of the certain motion of the object by comparing based on the comparison result.

2. The motion analysis device according to claim 1, further comprising
   motion frequency region storing section configured to store a range of motion frequency which can be obtained regarding the certain motion of the object,
   wherein the waveform analyzing section analyzes the certain physical amount by using the region information of motion frequency stored in the motion frequency region storing section.

3. The motion analysis device according to claim 1 or 2, wherein
   the motion analysis section uses the spectral intensity analysis result in a range of the motion frequency among the spectral intensity analysis result in each frequency analyzed by the waveform analyzing section as a subject to be compared and analyzes the motion pace of the certain motion of the object based on the comparison result.

**4.** The motion analysis device according to one of claims 1 to 3, wherein
the motion analysis section analyzes a physical amount related to a load of the certain motion of the object by using the spectral intensity analysis result of the frequency corresponding to the motion pace.

**5.** The motion analysis device according to one of claims 1 to 4, wherein
the motion analysis section determines whether an abnormal action faster than a certain speed in the certain motion of the object occurs by using at least the spectral intensity analysis result in each frequency analyzed by the waveform analyzing section.

**6.** The motion analysis device according to one of claims 1 to 5, wherein
the certain function used by the waveform analyzing section is a function of a mother wavelet in wavelet transform and a parameter related to a scale corresponding to the frequency is applied to the function of the mother wavelet.

**7.** The motion analysis device according to one of claims 1 to 6, wherein
the certain function used by the waveform analyzing section is a function of a mother wavelet in wavelet transform and a parameter related to a scale corresponding to the frequency is applied to the function of the mother wavelet.

**8.** A motion analysis program that controls a computer mounted in a motion analysis device constituting a motion analysis system that includes a sensor device for measuring a motion amount related to motion of an object which repeats a certain motion and the motion analysis device for analyzing the certain motion of the object based on the measured result by the sensor device,
the motion analysis program that controls the computer to function as:

time series data storing section configured to store time series data of the motion amount measured by the sensor device;
waveform analyzing section configured to analyze the spectral intensity in each frequency by applying a certain function to a waveform shown by the time series data and analyze by applying different parameters for each frequency to the function; and
motion analysis section configured to compare, for different frequencies, the spectral intensity analysis result in each frequency analyzed by the waveform analyzing section and analyze a certain physical amount of the certain motion of the object by comparing based on the comparison result.

**9.** A motion analysis method for analyzing a certain motion of an object based on a measured result by a sensor device for measuring a motion amount related to the motion of the object which repeats the certain motion, comprising
time series data storing section, waveform analyzing section and motion analysis section, wherein
the time series data storing section stores time series data of the motion amount measured by the sensor device,
the waveform analyzing section analyzes the spectral intensity in each frequency by applying a certain function to a waveform shown by the time series data and analyzes by applying different parameters for each frequency to the function, and
the motion analysis section compares, for different frequencies, the spectral intensity analysis result in each frequency analyzed by the waveform analyzing section and analyzes a certain physical amount of the certain motion of the object by comparing based on the comparison result.

**10.** A motion analysis system that includes a sensor device for measuring a motion amount related to motion of an object which repeats a certain motion and the motion analysis device for analyzing the certain motion of the object based on the measured result by the sensor device, wherein
the motion analysis device according to claim 1 is applied as the motion analysis device.

**11.** The method of claim 9, further comprising the method steps as carried out by the features of the motion analysis device as additionally defined in one of claims 2 to 7.

**12.** The motion analysis program of claim 8, further comprising the features as additionally defined in one of claims 2 to 7.

EP 2 250 964 A1

**FIG.1**

<u>1</u>  MOTION ANALYSIS SYSTEM

# FIG.2

| MOTION CATEGORY | MINIMUM FREQUENCY | MAXIMUM FREQUENCY |
|---|---|---|
| RUNNING | 2.0Hz | 3.5Hz |
| RIDING BICYCLE | 1.0Hz | 5.0Hz |
| WALKING | 1.5Hz | 2.2Hz |
| WEIGHT TRAINING | 0.1Hz | 0.5Hz |
| ⋮ | | |

# FIG.3

```
        START
          │
          ▼
      SENSING        ～S101
          │
          ▼
   PREPROCESSING     ～S102
          │
          ▼
   CONVERT DATA      ～S103
          │
          ▼
     OBTAIN          ～S104
  CONVERTED DATA
          │
          └──────────────────┐
                             ▼
                        CALCULATE
                        CUMULATIVE    ～S105
                        INTENSITY
                             │
                             ▼
                       SELECT BASIS   ～S106
                        FUNCTION
                             │
                             ▼
                      OUTPUT RESULT   ～S107
                             │
                             ▼
                           END
```

FIG.4

# FIG.5

# FIG.6

# FIG.7

# FIG.8

# FIG.9

<u>1A</u>  MOTION ANALYSIS SYSTEM

**FIG.10**

| EXPLANATORY VARIABLE | | | OBJECTIVE VARIABLE (MOTION AMOUNT) | |
|---|---|---|---|---|
| TYPE OF ACTION | MOTION PACE (Hz) | MOTION PACE SPECTRUM (dB) | LOAD OF EQUIPMENT (kg) | CONSUMED CALORIES (cal/sec) |
| WEIGHT TRAINING | 0.39 | 3.52 | 15.5 | 4.78 |
| WEIGHT TRAINING | 0.23 | 3.68 | 26.0 | 5.98 |
| WEIGHT TRAINING | 0.28 | 3.75 | 38.5 | 8.37 |
| WEIGHT TRAINING | 0.31 | 3.49 | 22.5 | 5.50 |
| WEIGHT TRAINING | 0.17 | 3.80 | 42.0 | 9.72 |
| SLOW WALKING | 2.33 | 3.62 | — | 3.11 |
| SLOW WALKING | 2.54 | 3.25 | — | 2.63 |
| FAST WALKING | 2.86 | 2.89 | — | 5.26 |
| FAST WALKING | 3.23 | 3.12 | — | 6.45 |
| ⋮ | | | | |

## FIG.11

# FIG.12

```
        ┌─────────┐
        │  START  │
        └─────────┘
             │
             ▼
     ┌───────────────┐
     │    SENSING    │───S201
     └───────────────┘
             │
             ▼
     ┌───────────────┐
     │ PREPROCESSING │───S202
     └───────────────┘
             │
             ▼
     ┌───────────────┐
     │ CONVERT DATA  │───S203
     └───────────────┘
             │
             ▼
     ┌───────────────┐
     │    OBTAIN     │───S204
     │CONVERTED DATA │
     └───────────────┘
```

```
     ┌───────────────┐
     │   CALCULATE   │
     │  CUMULATIVE   │───S205
     │   INTENSITY   │
     └───────────────┘
             │
             ▼
     ┌───────────────┐
     │  SELECT BASIS │───S206
     │   FUNCTION    │
     └───────────────┘
             │
             ▼
     ┌───────────────┐
     │ CONVERT MOTION│───S207
     │    AMOUNT     │
     └───────────────┘
             │
             ▼
     ┌───────────────┐
     │ OUTPUT RESULT │───S208
     └───────────────┘
             │
             ▼
        ┌─────────┐
        │   END   │
        └─────────┘
```

## FIG.13

1B MOTION ANALYSIS SYSTEM

SENSING DEVICE ~10

SENSING UNIT ~11

COMMUNICATION CONTROL UNIT ~12

MOTION ANALYSIS DEVICE 20B

RANGE INFORMATION STORAGE UNIT ~23

CALCULATING UNIT 24B

ABNORMAL ACTION DETECTION UNIT 28

OUTPUT UNIT 25

DATA ACCUMULATION UNIT ~22

COMMUNICATION CONTROL UNIT ~21

EP 2 250 964 A1

# FIG.14

# FIG.15

# FIG.16

# FIG.17

**FIG.18**

**EUROPEAN SEARCH REPORT**

Europäisches
Patentamt
European
Patent Office
Office européen
des brevets

Application Number

EP 10 16 1185

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/139692 A1 (BARREY ERIC [FR] ET AL) 24 July 2003 (2003-07-24)<br>* paragraph [0002] *<br>* paragraphs [0100] - [0117] *<br>* paragraphs [0134] - [0172] *<br>* figures 9-17 *<br>----- | 1-12 | INV.<br>A61B5/11<br>A63B24/00<br>A61B5/103 |
| X | WO 2009/023923 A1 (COMMW SCIENT IND RES ORG [AU]; BIDARGADDI NIRANJAN [AU]; SARELA ANTTI) 26 February 2009 (2009-02-26) | 1-4,6-12 | |
| A | * paragraph [0032] *<br>* paragraphs [0047] - [0057] *<br>----- | 5 | |
| X | US 6 571 193 B1 (UNUMA MUNETOSHI [JP] ET AL) 27 May 2003 (2003-05-27)<br>* column 7, line 36 - column 11, line 13 *<br>* column 14, line 45 - column 5, line 12 *<br>----- | 1-12 | |
| X | US 2008/001735 A1 (TRAN BAO [US]) 3 January 2008 (2008-01-03)<br>* paragraphs [0384] - [0401] *<br>----- | 1-12 | TECHNICAL FIELDS SEARCHED (IPC)<br><br>A61B<br>A63B |

The present search report has been drawn up for all claims

| Place of search | Date of completion of the search | Examiner |
|---|---|---|
| The Hague | 31 August 2010 | Dydenko, Igor |

EPO FORM 1503 03.82 (P04C01)

## ANNEX TO THE EUROPEAN SEARCH REPORT
## ON EUROPEAN PATENT APPLICATION NO.

EP 10 16 1185

This annex lists the patent family members relating to the patent documents cited in the above-mentioned European search report.
The members are as contained in the European Patent Office EDP file on
The European Patent Office is in no way liable for these particulars which are merely given for the purpose of information.

31-08-2010

| Patent document cited in search report | | Publication date | Patent family member(s) | | Publication date |
|---|---|---|---|---|---|
| US 2003139692 | A1 | 24-07-2003 | AT 372089 T | | 15-09-2007 |
| | | | AU 3560901 A | | 14-08-2001 |
| | | | CA 2399182 A1 | | 09-08-2001 |
| | | | DE 60130325 T2 | | 29-05-2008 |
| | | | DK 1261279 T3 | | 02-01-2008 |
| | | | EP 1261279 A1 | | 04-12-2002 |
| | | | ES 2292560 T3 | | 16-03-2008 |
| | | | FR 2804596 A1 | | 10-08-2001 |
| | | | WO 0156470 A1 | | 09-08-2001 |
| | | | PT 1261279 E | | 06-12-2007 |
| WO 2009023923 | A1 | 26-02-2009 | AU 2008288697 A1 | | 26-02-2009 |
| US 6571193 | B1 | 27-05-2003 | NONE | | |
| US 2008001735 | A1 | 03-01-2008 | NONE | | |

**REFERENCES CITED IN THE DESCRIPTION**

**Patent documents cited in the description**

- JP 2006118909 A
- JP 2005233771 A
- JP 2005342254 A
- JP 2004121539 A
- JP 2005049202 A
- JP 2008154733 A
- JP 2006101973 A
- JP 2009115720 A